(19) **Europäisches Patentamt**
**European Patent Office**
**Office européen des brevets**

(11) **EP 2 266 453 A1**

(12) **EUROPEAN PATENT APPLICATION**

(43) Date of publication:
**29.12.2010 Bulletin 2010/52**

(51) Int Cl.:
***A61B 1/005*** *(2006.01)*

(21) Application number: **10165665.0**

(22) Date of filing: **11.06.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **12.06.2009 JP 2009141196**

(71) Applicant: **Fujifilm Corporation**
**Minato-ku**
**Tokyo (JP)**

(72) Inventors:
• **ENDO, Azuchi**
**Kanagawa-ken (JP)**

• **KURODA, Osamu**
**Kanagawa-ken (JP)**
• **NAKAMURA, Takayuki**
**Kanagawa-ken (JP)**
• **IDA, Takayuki**
**Kanagawa-ken (JP)**
• **OZAKI, Takao**
**Kanagawa-ken (JP)**

(74) Representative: **Klunker . Schmitt-Nilson . Hirsch**
**Patentanwälte**
**Destouchesstrasse 68**
**80796 München (DE)**

(54) **Endoscope system, endoscope, and driving method**

(57) An endoscope (10) includes an elongated tube (13). An objective lens system (30) is disposed in the elongated tube, for passing image light from an object (80). A fiber optic image guide (31) includes plural optical fibers (52) bundled together, has a distal tip (48), is inserted through the elongated tube, for transmitting the image light focused on the distal tip by the objective lens system in a proximal direction. A displacing device (32, 90, 100) displaces the distal tip laterally and periodically upon receiving entry of the image light being focused by use of a piezoelectric actuator (35, 92) positioned outside the distal tip. An image sensor (56) creates images in plural set positions of the distal tip. First to fourth images among the plural images created consecutively are combined to form a first synthesized image. Then second to fifth images among the plural images without use of the first image are combined to form a second synthesized image.

FIG. 3

EP 2 266 453 A1

## Description

BACKGROUND OF THE INVENTION

1. Field of the Invention

The present invention relates to an endoscope system, endoscope, and driving method. More particularly, the present invention relates to an endoscope system, endoscope, and driving method in which an elongated tube of the endoscope can have a reduced diameter and images of high image quality can be produced even with a frame rate of a widely used high value.

2. Description Related to the Prior Art

An endoscope is an important medical instrument used in the field of the clinical medicine. Examples of the endoscope include primitive models such as a fiberscope or stomach camera, an electronic endoscope containing a CCD, and a capsule endoscope which is orally swallowed by a patient to retrieve an image.

In the field of endoscopic examination, extreme thinning of a tube for a reduced diameter to produce an ultra thin tube is desired very seriously for an elongated tube of the endoscope. Various ideas for the extreme thinning for a reduced diameter have been suggested for the purpose of imaging of various body parts in a narrow lumen, such as a pancreatic duct, bile duct, breast duct, terminal bronchioles, and the like.

The fiberscope is structurally suitable for the extreme thinning for a reduced diameter, because the image can be retrieved only by having a fiber optic image guide and an illuminating light guide. The fiber optic image guide transmits image light of the image of a body part or object of interest. The illuminating light guide applies light to the body part. However, cladding of an optical fiber bundle constituting the fiber optic image guide does not contribute to the transmission of the image light. There occurs a problem in that a pattern of mesh of a loss region due to the cladding appears locally within the image, and the image quality of the image will be low.

In view of this problem, U.S.P. No. 4,618,884 (corresponding to JP-A 60-053919) discloses the fiberscope. In a first embodiment of the document, a focusing lens system is disposed at a distal tip of the fiber optic image guide for focusing of image light on the distal tip. A piezoelectric actuator vibrates the focusing lens system to remove light component of a pattern of mesh from the image. The piezoelectric actuator vibrates the focusing lens system horizontally and vertically at a predetermined amount according to a pixel pitch of the pixels of the CCD or the optical fibers in the fiber optic image guide.

In a second embodiment of U.S.P. No. 4,618,884, the CCD is disposed at a distal end of the elongated tube without the fiber optic image guide. The focusing lens system in front of the CCD is vibrated in the same manner as its first embodiment. During the vibration, image light of the image is received on pixels of the CCD in a time division manner. Data are obtained, and written to a frame memory sequentially, to produce one frame of the image. Thus, high definition of the image can be obtained.

The focusing lens system has a larger diameter than the fiber optic image guide to ensure high brightness in the image. In U.S.P. No. 4, 618, 884, the focusing lens system is vibrated by the piezoelectric actuator. Even with the focusing lens system having the larger diameter than the fiber optic image guide, a further space is required for positioning a frame or retention mechanism for supporting the focusing lens system in a pivotally movable manner. The elongated tube must have a larger size in the radial direction. It follows that vibrating the focusing lens system with the piezoelectric actuator is inconsistent to the extreme thinning for a reduced diameter. A space required for positioning such a frame or retention mechanism is a serious problem in view of the extreme thinning for a reduced diameter of an order from tens of microns to a number of millimeters.

Although high definition of an image can be obtained from the second embodiment of U.S.P. No. 4,618,884, the extreme thinning for a reduced diameter is still impossible because the CCD is disposed in front of the elongated tube in addition to the focusing lens system. Also, there is a problem in that only an image of one frame can be produced from plural images obtained in one shift sequence of vibration of the focusing lens system, to lower a frame rate of a moving image. A frequency of a clock signal to drive the CCD may be raised to solve such a problem, but will require very expensive elements of hardware.

SUMMARY OF THE INVENTION

In view of the foregoing problems, an object of the present invention is to provide an endoscope system, endoscope, and driving method in which an elongated tube of the endoscope can have a reduced diameter and images of high image quality can be produced even with a frame rate of a widely used high value.

In order to achieve the above and other objects and advantages of this invention, an endoscope system includes an objective lens system, disposed in an elongated tube of an endoscope, for passing image light from an object. A fiber optic image guide includes plural optical fibers bundled together, has a distal tip, inserted through the elongated tube, for transmitting the image light focused on the distal tip by the objective lens system in a proximal direction. An image sensor detects the image light from the fiber optic image guide. A displacing device displaces the distal tip laterally and periodically upon receiving entry of the image light being focused by use of a piezoelectric actuator positioned outside the distal tip. A sync control unit drives the image sensor for detecting the image light for plural times in synchronism with displacement of the displacing device, and controls the image sensor and the displacing device to create plural images in plural set positions of the distal tip relative to the image light being focused. An image synthesizing unit combines first to Nth images being N of the plural images created consecutively to form a first synthesized image, and then combines second to (N+1)th images being N of the plural images without use of the first image to form a second synthesized image, to output plural synthesized images consecutively by repeating forming of the first and second synthesized images, where N is an integer of two or more.

The displacing device shifts the distal tip stepwise from a first set position to an Nth set position, and shifts back the distal tip to the first set position for one two-dimensional shift sequence. The image sensor carries out image pickup for each of the first to Nth set positions.

The displacing device retains the distal tip in each of the first to Nth set positions by intermittent shifting.

A distance between the first to Nth set positions is 1/n as long as a pitch of arrangement of the optical fibers in the fiber optic image guide, wherein n is an integer.

N is 4 or 9, and the first to Nth set positions are arranged in a rhombic shape in which two or three of the set positions are arranged on one edge thereof and which has interior angles of substantially 60 and 120 degrees.

The displacing device shifts the distal tip with a shortest path length defined by arranging the first to Nth set positions.

The first to Nth set positions are arranged on a polygonal path.

The sync control unit controls a plurality of the piezoelectric actuator in a predetermined sequence.

Furthermore, a laser light source supplies the light guide devices with laser light. A wavelength conversion device is disposed at a distal end of the light guide devices, for emitting light by excitation with the laser light, to obtain white light by mixing the excitation light with the laser light.

Each of the optical fibers includes a core, and a cladding disposed about the core. The displacing device shifts the distal tip at a shift amount for setting a distal end of the core at a location where a distal end of the cladding has been set.

Also, an endoscope includes an elongated tube. An objective lens system is disposed in the elongated tube, for passing image light from an object. A fiber optic image guide includes plural optical fibers bundled together, has a distal tip, inserted through the elongated tube, for transmitting the image light focused on the distal tip by the objective lens system in a proximal direction. A displacing device displaces the distal tip laterally and periodically upon receiving entry of the image light being focused by use of a piezoelectric actuator positioned outside the distal tip. A support casing supports the distal tip inserted therein, keeps the distal tip shiftable on the displacing device, and transmits force of the piezoelectric actuator disposed outside to the fiber optic image guide. The fiber optic image guide transmits the image light to an image sensor for detecting the image light for plural times in synchronism with displacement of the displacing device, the image sensor and the displacing device are controlled to create plural images in plural set positions of the distal tip relative to the image light being focused. First to Nth images among the plural images created consecutively are combined to form a first synthesized image, and then second to (N+1)th images among the plural images are combined without use of the first image to form a second synthesized image, to output plural synthesized images consecutively by repeating forming of the first and second synthesized images, where N is an integer.

The support casing is substantially cylindrical.

In a preferred embodiment, the support casing is substantially in a shape of a quadrilateral prism.

An electrode of the piezoelectric actuator is constituted by the support casing.

The displacing device includes a stationary section secured inside the elongated tube. A shift mechanism is disposed to extend from the stationary section in a distal direction, for displacing the distal tip.

The piezoelectric actuator extends fully along the displacing device inclusive of the stationary section and the shift mechanism. An electrode of the piezoelectric actuator extends to a proximal end of the stationary section.

Furthermore, a regulating portion is formed with any one of the displacing device and an inner surface of the elongated tube, for regulating an orientation of the displacing device.

Also, a driving method of driving an endoscope is provided, the endoscope includes an elongated tube, an objective lens system, disposed in the elongated tube, for passing image light from an object, and a fiber optic image guide, including plural optical fibers bundled together, having a distal tip, inserted through the elongated tube, for transmitting the image light focused on the distal tip by the objective lens system in a proximal direction. In the driving method, the distal tip is displaced laterally and periodically upon receiving entry of the image light being focused by use of a piezoelectric actuator positioned outside the distal tip. An image sensor is driven for detecting the image light for plural times in synchronism with displacement, to create plural images with the image sensor in plural set positions of the distal tip relative to the image light being focused. First to Nth images among the plural images created consecutively are combined to form a first synthesized image, and then second to (N+1)th images among the plural images are combined without use of the first image to form a second synthesized image, to output plural synthesized images consecutively by repeating forming of the first and second synthesized images, where N is an integer.

Consequently, images of high image quality can be produced even with a frame rate of a widely used high value, because synthesized images can be serially formed at the frame rate equal to that of original images created consecutively on the basis of the plural set positions of the distal tip of the fiber optic image guide.

BRIEF DESCRIPTION OF THE DRAWINGS

The above objects and advantages of the present invention will become more apparent from the following detailed description when read in connection with the accompanying drawings, in which:

Fig. 1 is a perspective view illustrating an endoscope system;
Fig. 2 is a front elevation illustrating a head assembly of an endoscope of the endoscope system;
Fig. 3 is a vertical section illustrating the head assembly;
Fig. 4 is a perspective view illustrating a displacing device;
Fig. 5 is a front elevation illustrating a bundle of optical fibers of a fiber optic image guide;
Fig. 6 is a block diagram illustrating relevant elements in the endoscope system;
Fig. 7 is an explanatory view in a front elevation illustrating a relationship between an image transmitted by the core and a pixel of a CCD;
Fig. 8 is an explanatory view illustrating one example of displacement;
Fig. 9A is an explanatory view in a front elevation illustrating a two-dimensional path of a distal tip of one of the cores;
Fig. 9B is an explanatory view in a front elevation illustrating another two-dimensional path of the distal tip;
Fig. 10 is a block diagram illustrating relevant circuits for operation upon designating a composite imaging mode;
Fig. 11 is a timing chart illustrating a relationship between driving of the CCD, a piezoelectric control signal and an image synthesis signal;
Fig. 12 is a flow chart illustrating operation of the endoscope system;
Fig. 13 is a front elevation illustrating a head assembly of another preferred endoscope;
Fig. 14 is a perspective view illustrating one example of displacement;
Fig. 15 is an explanatory view illustrating another example of displacement;
Fig. 16 is an explanatory view illustrating another example of the displacing;
Fig. 17A is an explanatory view in a front elevation illustrating a two-dimensional path of a distal tip of one of the cores;
Fig. 17B is an explanatory view in a front elevation illustrating one preferred two-dimensional path of the distal tip;

Fig. 17C is an explanatory view in a front elevation illustrating still another preferred two-dimensional path of the distal tip;
Fig. 18 is a front elevation illustrating one preferred displacing device having a regulating recess inside;
Fig. 19 is a front elevation illustrating one preferred embodiment having a regulating projection;

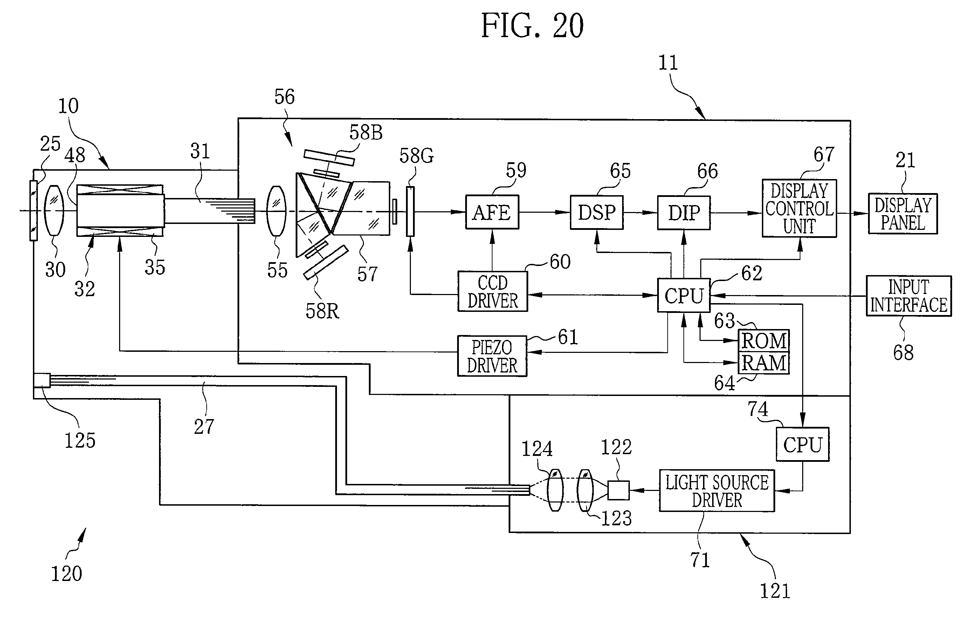

Fig. 20 is a block diagram illustrating another preferred embodiment having a laser light source.

DETAILED DESCRIPTION OF THE PREFERRED EMBODIMENT(S) OF THE PRESENT INVENTION

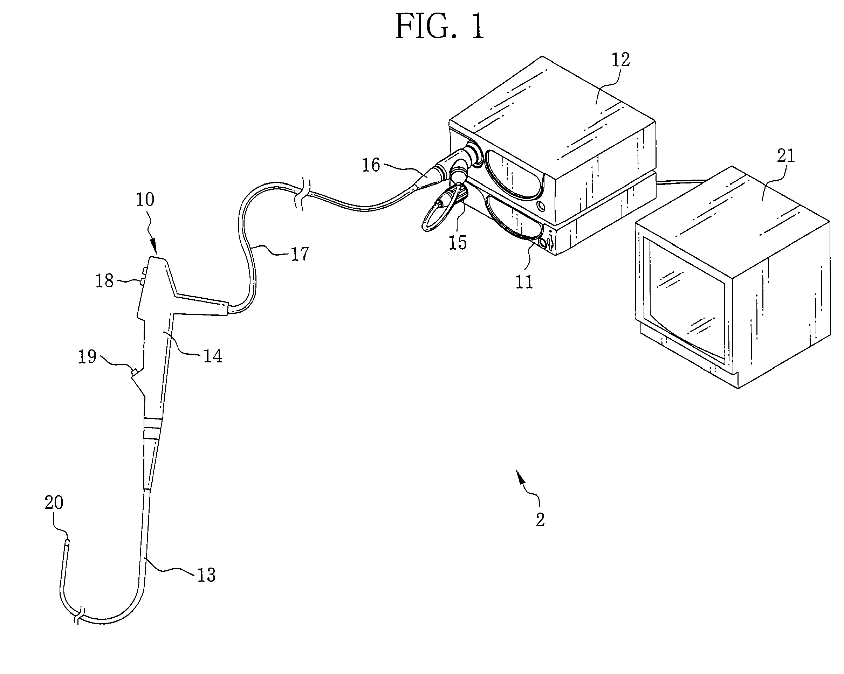

In Fig. 1, an endoscope system 2 includes an endoscope 10, a processing apparatus 11 and a light source apparatus 12. The endoscope 10 is used for imaging of various body parts in narrow lumens, for example, a pancreatic duct, bile duct, breast duct, terminal bronchioles, and the like. The endoscope 10 includes an elongated tube 13 or insertion tube, a handle 14, a first connector 15, a second connector 16 or coupler, and a universal cable 17. The elongated tube 13 is flexible and entered in a patient's body. The handle 14 is disposed at a proximal end of the elongated tube 13. The first connector 15 is plugged in the processing apparatus 11. The second connector 16 is plugged in the light source apparatus 12. The universal cable 17 extends from the handle 14 to the first connector 15 and the second connector 16.

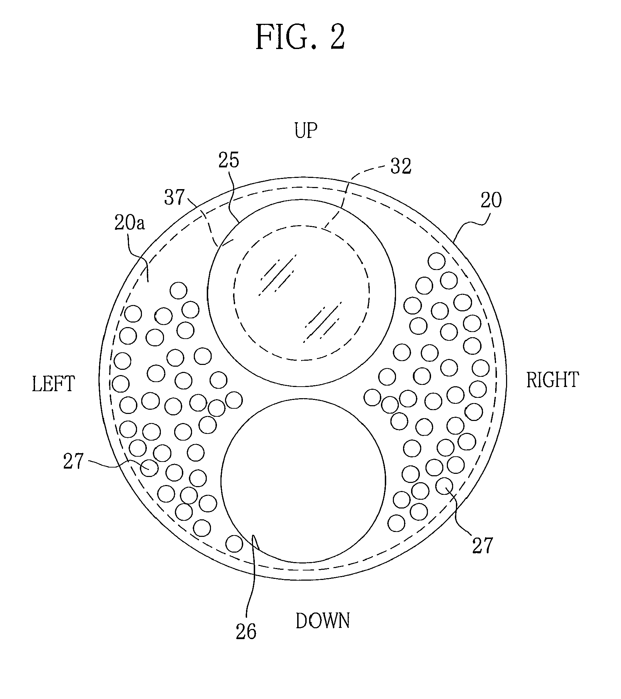

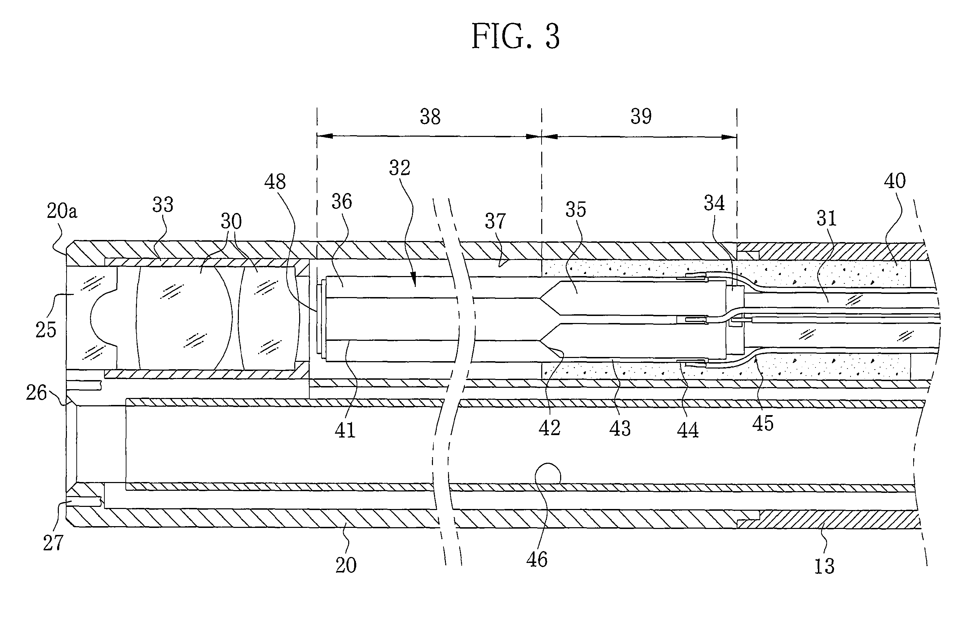

The elongated tube 13 has a thickness of 50 microns and outer diameter of 0.9 mm, and is formed from flexible material such as a Teflon (trade name), namely tetrafluoroethylene. A recording button 18 or release button among various buttons is disposed on the handle 14 for recording an endoscopic still image of a body part. An instrument opening 19 or forceps opening is formed on a side of the handle 14, and receives passage of an electrosurgical knife or other instruments for treatment. A head assembly 20 is disposed at a distal end of the elongated tube 13. An instrument opening 26 or forceps opening (See Fig. 2) is formed in the head assembly 20. A working channel 46 or forceps channel (See Fig. 3) is formed through the elongated tube 13, and extends from the instrument opening 19 to the instrument opening 26.

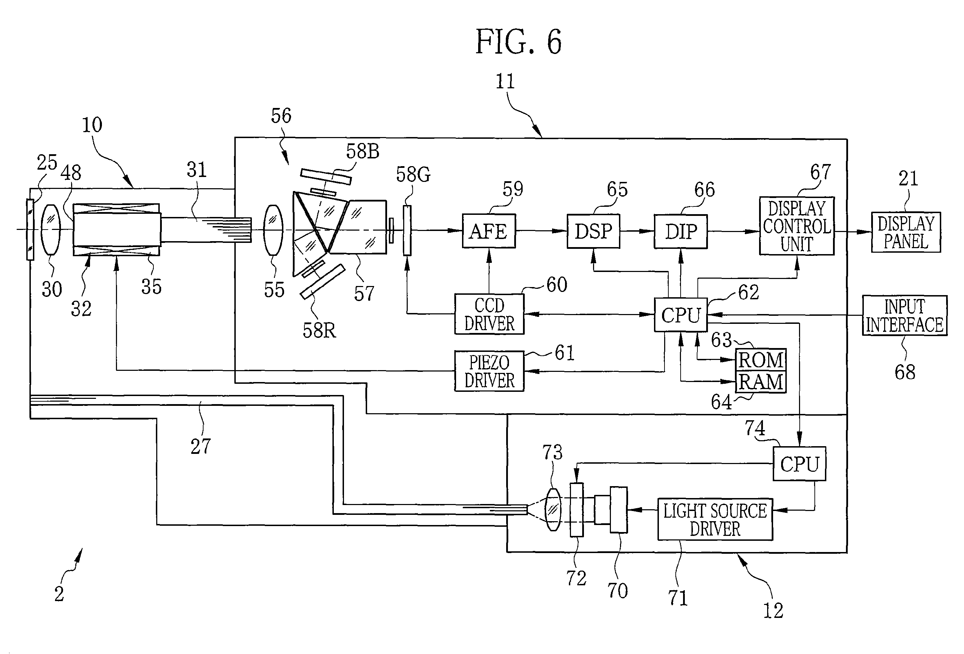

The processing apparatus 11 is connected with the light source apparatus 12 electrically, and controls operation of the constituents of the endoscope system 2. A connection cable 45 of Fig. 3 is inserted through the universal cable 17 and the elongated tube 13, and supplies the endoscope 10 with power from the processing apparatus 11. A displacing device 32 of Fig. 3 is also controlled by the processing apparatus 11. A fiber optic image guide 31 and a CCD group 58 are contained in the processing apparatus 11. The CCD group 58 includes CCDs 58B, 58G and 58R or image pickup devices. See Fig. 6. Image light of an image of a body part is transmitted by the fiber optic image guide 31, and received by the CCD group 58, which generates an image signal. The processing apparatus 11 processes the image signal in image processing, to create an image. A monitor display panel 21 is connected by use of a cable, and displays the image created by the processing apparatus 11.

The head assembly 20 has a wall constituted by a pipe of a stainless steel and has a thickness of 25 microns and an outer diameter of 0.8 mm. In Fig. 2, a distal end face 20a of the head assembly 20 is illustrated. An imaging window 25 is disposed in an upper portion of the distal end face 20a. The instrument opening 26 is open in the distal end face 20a and disposed under the imaging window 25. A plurality of light guide devices 27 or illumination fiber optics are contained in the head assembly 20. Ends of the light guide devices 27 are positioned beside the imaging window 25 and the instrument opening 26 without bundling and packed randomly in a tube lumen inside the head assembly 20.

The instrument opening 26 has an outer diameter of 0.34 mm and an inner bore of 0.3 mm, and is an exit opening of the working channel 46. See Fig. 3. An example of material of a wall of the working channel 46 is polyimide. An example of the light guide devices 27 is an optical fiber having a diameter of 50 microns. The light guide devices 27 are inserted through the elongated tube 13 and the universal cable 17, and have a proximal end located in the second connector 16 or coupler. Light is entered through the proximal end of the light guide devices 27, is transmitted, and is applied to an object of interest through a distal end of the light guide devices 27 positioned within the distal end face 20a.

For the light guide devices 27, a plurality of optical fibers without bundling are inserted in the elongated tube 13. Adhesive agent in a fluid form is supplied into the head assembly 20 for adhesion of the light guide devices 27. It is possible according to requirements to polish the surface of the distal end of the light guide devices 27 after the adhesion, or to dispose a lighting window in front of the distal end of the light guide devices 27 to cover the same. Also, a coating of phosphor or other materials may be applied to the lighting window to diffuse the light.

In Fig. 3, an objective lens system 30 is disposed behind the imaging window 25 together with the fiber optic image guide 31 and the displacing device 32. The displacing device 32 shifts the fiber optic image guide 31. A lens barrel 33 contains the objective lens system 30. A distal tip 48 for receiving light is positioned on a plane where image light of an image from an object is in focus. A diameter of the objective lens system 30 is 0.35 mm. An outer diameter of the lens barrel 33 is 0.4 mm. A length of the lens barrel 33 in the axial direction is 3.2 mm.

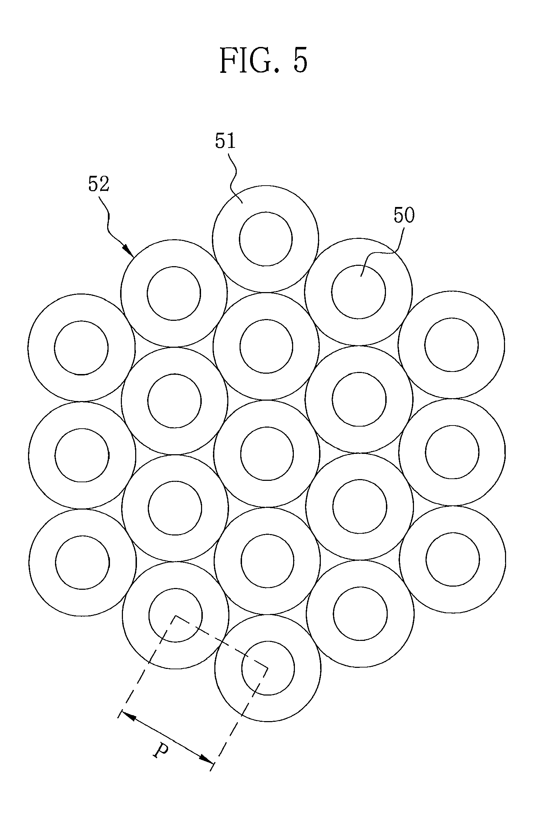

The fiber optic image guide 31 is a bundle of optical fibers with a diameter of 0.2 mm. See Fig. 5. The fiber optic image guide 31 extends through the elongated tube 13 and the universal cable 17, and has a proximal tip contained in the first connector 15. The fiber optic image guide 31 transmits image light of an object received from the objective lens system 30 through the distal tip 48 toward its proximal tip.

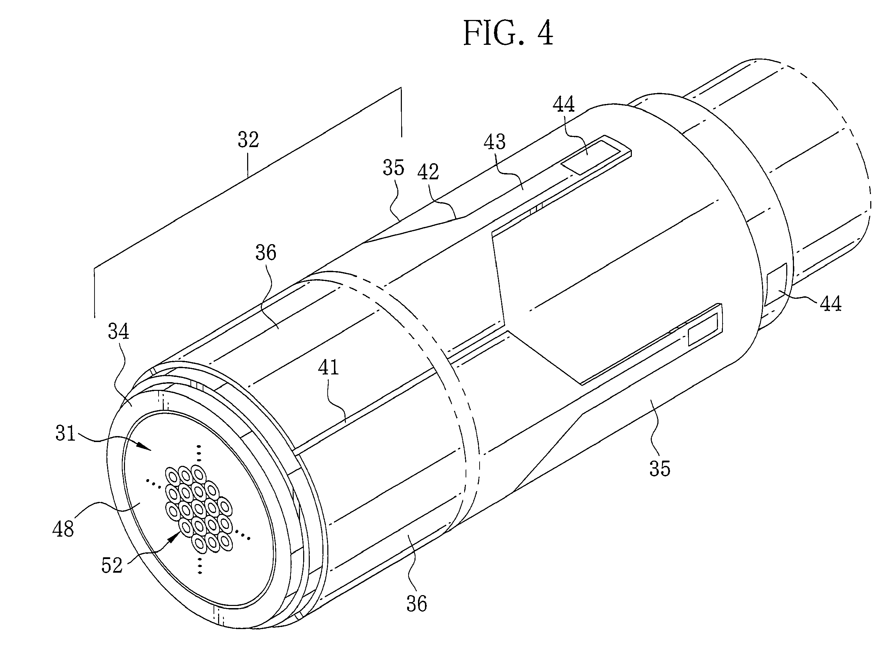

In Fig. 4, the displacing device 32 includes a support casing 34, a piezoelectric actuator material 35 and electrodes 36. The support casing 34 is a barrel or pipe of stainless steel, and has an outer diameter of 0.26 mm and an inner bore of 0.2 mm. The fiber optic image guide 31 is inserted in and fixed on the support casing 34. The piezoelectric actuator material 35 has a thickness of 15 microns, and is a coating applied to an outer surface of the support casing 34 in a cylindrical form. The electrodes 36 have a thickness of 5 microns, and are a coating about the piezoelectric actuator material 35.

The displacing device 32 is contained in a wall of the head assembly 20. A lumen 37 is defined between the outside of the displacing device 32 and the inside of the wall of the head assembly 20, and has a width of approximately 0.1 mm.

The displacing device 32 includes a shift mechanism 38 and a stationary section 39. The shift mechanism 38 is a portion of the displacing device 32 free from the wall of the head assembly 20 without fixing. The fiber optic image guide 31 is displaceable within the lumen 37 with respect to the stationary section 39. Adhesive agent 40 is used in the stationary section 39, and attaches the displacing device 32 to the inner wall of the head assembly 20. An area of the adhesive agent 40 extends from a proximal point of the displacing device 32 where the fiber optic image guide 31 appears to a point near to a distal end of the elongated tube 13. Lengths of the shift mechanism 38 and the stationary section 39 are respectively 4 mm and 1.9 mm in the axial direction. A length of filling of the adhesive agent 40 in the axial direction is 3.2 mm inclusive of the stationary section 39 and a distal portion of the elongated tube 13.

The electrodes 36 are arranged in the circumferential direction regularly at an angle of 90 degrees. The electrodes 36 are oriented with an inclination of 45 degrees relative to the vertical or horizontal direction of Fig. 2. Four grooves 41 are formed to extend in parallel with the axial direction, and define the electrodes 36 of two pairs. The electrodes 36 have a locally large width in the shift mechanism 38, as an interval between the electrodes 36 is only as great as the width of the grooves 41. In contrast, recesses 42 are defined with the electrodes 36 in the area of the stationary section 39, and extend in a symmetrical manner from the grooves 41. A narrow portion 43 of the electrodes 36 is defined by the recesses 42. The narrow portion 43 extends to the vicinity of the proximal end of the piezoelectric actuator material 35. The grooves 41 and the recesses 42 are formed by etching after applying a coating of the electrode material to the entire surface of the piezoelectric actuator material 35.

Pads 44 are disposed at proximal ends of the narrow portion 43. The connection cable 45 is connected with each of the pads 44. Also, the end of the support casing 34 has another one of the pads 44, with which the connection cable 45 is connected. Consequently, the support casing 34 operates as a common electrode for the piezoelectric actuator material 35.

The connection cable 45 has a cable diameter of 15 microns and an outer jacket diameter of 20 microns. The connection cable 45 is extended about the fiber optic image guide 31, inserted in the elongated tube 13 and the universal cable 17, and connected by the first connector 15 with the processing apparatus 11.

The two pairs of the electrodes 36 are supplied with voltages of opposite polarities with reference to a voltage applied to the support casing 34 as a common electrode. For example, let the support casing 34 have a potential of 0 V. An upper one of the electrodes 36 is supplied with +5 V. A lower one of the electrodes 36 is supplied with -5 V. The piezoelectric actuator material 35 under the electrodes 36 expands and contracts axially. In response to this, a portion of the shift mechanism 38 in front of the stationary section 39 displaces in the lumen 37 together with the distal tip 48 of the fiber optic image guide 31. It is possible to displace the shift mechanism 38 at a predetermined angle and amount by changing a combination of the electrodes 36 for powering and levels of the voltages.

In Fig. 5, the fiber optic image guide 31 has plural optical fibers 52, for example 6,000 fibers, bundled with extreme tightness in an equilateral hexagonal form. Each of the optical fibers 52 includes a core 50 and a cladding 51. A diameter of the core 50 is 3 microns. A diameter of the cladding 51 is 6 microns. A pitch P of arrangement of the optical fibers 52 is 6 microns.

In Fig. 6, the processing apparatus 11 includes an enlarging lens system 55 and a three-CCD assembly 56. The enlarging lens system 55 is opposed to the proximal tip of the fiber optic image guide 31 extending to the outside of the first connector 15. The enlarging lens system 55 enlarges the object image from the fiber optic image guide 31 with a suitable magnification, and directs its image light to the three-CCD assembly 56.

The three-CCD assembly 56 is an image sensor disposed behind the enlarging lens system 55. A color separation prism 57 is combined with the CCD group 58 to constitute the three-CCD assembly 56 as well-known in the art. The color separation prism 57 includes three prism blocks and two dichroic mirrors disposed on optical faces of the prism blocks. The color separation prism 57 separates image light of a body part from the enlarging lens system 55 into light components of red, blue and green colors, which are directed to the CCD group 58. The CCD group 58 outputs an image signal according to light amounts of the light components from the color separation prism 57. Note that a CMOS image sensor may be used instead of the CCD.

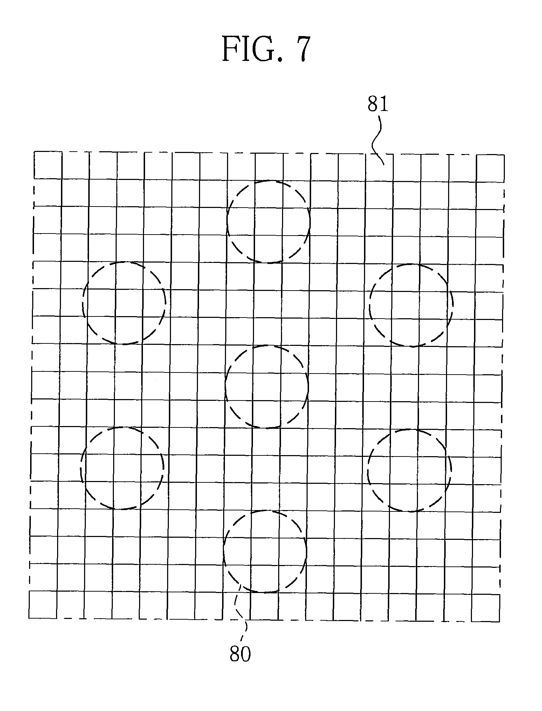

Image light of a part image 80 is transmitted by the core 50 of the fiber optic image guide 31. There are pixels 81 arranged on the image pickup surface of the CCD group 58. In Fig. 7, the part image 80 is viewed in a state projected to the image pickup surface of the CCD group 58. A center of the part image 80 substantially coincides with the center of a set of nine of the pixels 81. The proximal tip of the fiber optic image guide 31 is positioned relative to the color separation prism 57 and the CCD group 58 so as to correlate the part image 80 with the pixels 81 in the depicted state.

In Fig. 6, an analog front end 59 or AFE is supplied with the image signal from the CCD group 58. The analog front end 59 includes a correlated double sampling circuit or CDS circuit, an automatic gain control circuit or AGC circuit, and an A/D converter. The CDS circuit processes the image signal from the CCD group 58 in the correlated double sampling, and eliminates noise generated by the CCD group 58, such as reset noise, amplification noise and the like. The AGC circuit amplifies the image signal with a predetermined signal gain after noise elimination in the CDS circuit. The A/D converter converts the amplified image signal from the AGC circuit into a digital signal with a predetermined number of bits. A digital signal processor 65 or DSP has a frame memory (not shown) to which the digital form of the image signal from the A/D converter is written.

A CCD driver 60 generates drive pulses for the CCD group 58 and a sync pulse for the analog front end 59. The drive pulses include a vertical/horizontal scan pulse, electronic shutter pulse, reading pulse, reset pulse and the like. The CCD group 58 responds to the drive pulses from the CCD driver 60, takes an image, and outputs an image signal. Components included in the analog front end 59 are operated according to the sync pulse from the CCD driver 60. Note that the CCD driver 60 and the analog front end 59 are connected with the CCD 58G in the drawing, but also connected with the CCDs 58R and 58B.

A piezoelectric driver 61 is connected by the connection cable 45 with the electrodes 36 and the support casing 34. A CPU 62 controls the piezoelectric driver 61 to supply the piezoelectric actuator material 35 with voltage.

The CPU 62 controls the entirety of the processing apparatus 11. The CPU 62 is connected with various elements by a data bus (not shown), address bus, control lines and the like. A ROM 63 stores data (such as graphic data) and programs (operation system and application programs) for controlling the processing apparatus 11. The CPU 62 reads the program and data required for the purpose from the ROM 63. A RAM 64 is a working memory with which the CPU 62 performs tasks with data for operation by running the program. An input interface 68 is also associated with the CPU 62. The CPU 62 is supplied with information related to the examination by the input interface 68 or the LAN (local area network) or other networks, the information including a date and time of the examination, personal information of a patient, doctor's name, other text information, and the like. The CPU 62 writes the information to the RAM 64.

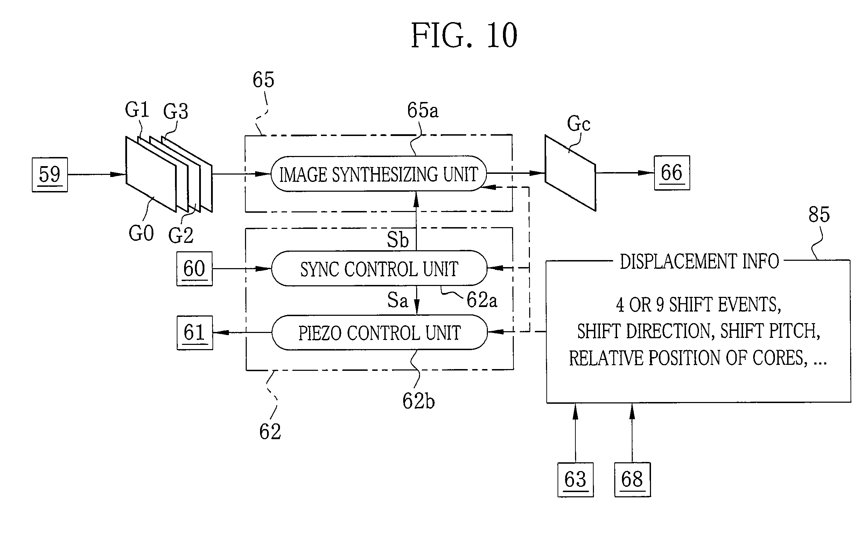

The digital signal processor 65 reads an image signal produced by the analog front end 59 from the frame memory. The digital signal processor 65 processes the image signal in processing of various functions, such as color separation, color interpolation, gain correction, white balance adjustment, gamma correction and the like, and produces an image of one frame. Also, the digital signal processor 65 has an image synthesizing unit 65a. See Fig. 10. When a composite imaging mode (to be described later) is selected, the image synthesizing unit 65a outputs one synthesized image of a high definition by combining plural images obtained in one two-dimensional shift sequence. To this end, plural frame memories are incorporated in the digital signal processor 65. A digital image processor 66 includes a frame memory (not shown), to which the image or synthesized image from the digital signal processor 65 is written.

The digital image processor 66 is controlled by the CPU 62 for image processing. The digital image processor 66 reads images from the frame memory after processing in the digital signal processor 65. Examples of functions of the image processing in the digital image processor 66 are electronic zooming, color enhancement, edge enhancement and the like. A display control unit 67 is supplied with data of the image processed by the digital image processor 66.

The display control unit 67 has a VRAM for storing the processed image from the digital image processor 66. The display control unit 67 receives graphic data read by the CPU 62 from the ROM 63 and the RAM 64. Examples of the graphic data include data of a mask for display of an active pixel area by masking an inactive area in the endoscopic image, text information such as an examination date, patient's name, and doctor's name, and data of graphical user interface (GUI), and the like. The display control unit 67 processes the image from the digital image processor 66 in various functions of display control, the functions including superimposition of the mask, the text information and the GUI, graphic processing of data for display on the display panel 21, and the like.

The display control unit 67 reads an image from the VRAM, and converts the image into a video signal suitable for display on the display panel 21, such as a component signal, composite signal or the like. Thus, the endoscopic image is displayed by the display panel 21.

The input interface 68 is a well-known input device, of which examples are an input panel on a housing of the processing apparatus 11, buttons on the handle 14 of the endoscope 10, mouse, keyboard, or the like. The CPU 62 operates relevant elements in the processing apparatus 11 in response to an input signal from the input interface 68.

The processing apparatus 11 also includes an image compression device, a media interface and a network interface. The image compression device compresses images in a format of compression, for example JPEG format. The media interface operates in response to an input from the recording button 18, and records the compressed images to a recording medium such as a CF card, MO (optical magnetic disk), CD-R and other removable media. The network interface transmits or receives various data by use of the LAN or other networks. Those are connected to the CPU 62 by a data bus or the like.

A light source 70 is incorporated in the light source apparatus 12. Examples of the light source 70 are a xenon lamp, white LED and the like which generate light of a broad band of the wavelength from red to blue, for example, with a wavelength of 480-750 nm. A light source driver 71 drives the light source 70. An aperture stop device 72 is disposed in front of the light source 70, and adjusts an amount of incident light. A condenser lens 73 condenses the light passed through the aperture stop device 72, and directs the light to the distal end of the light guide devices 27. A CPU 74 communicates with the CPU 62 of the processing apparatus 11, and controls the light source driver 71 and the aperture stop device 72.

There are two imaging modes including a normal imaging mode without operating the displacing device 32 and a composite imaging mode in operation of the displacing device 32. In the composite imaging mode, the number of shift events is changeable between four and nine. The input interface 68 can be operated to change over the imaging modes and set the number of shift events.

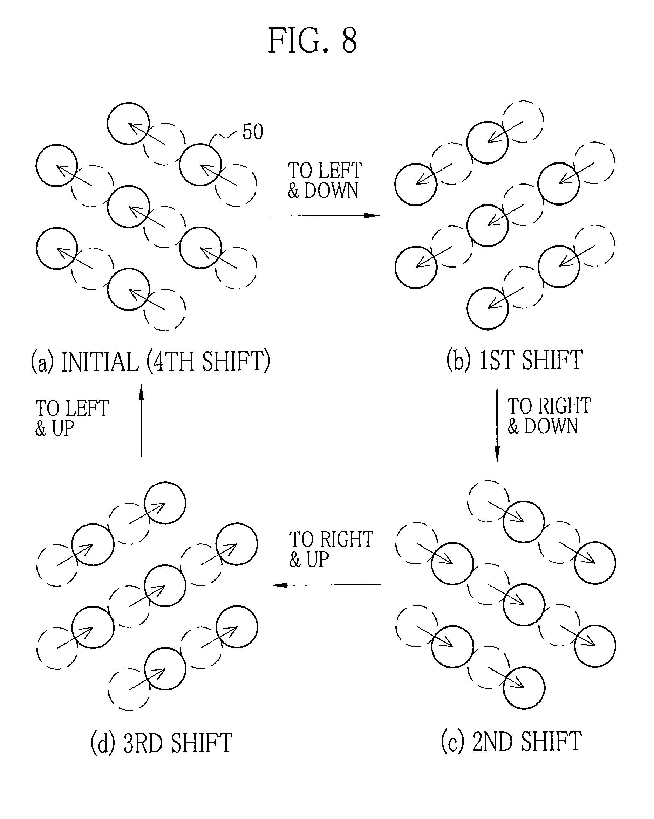

When the composite imaging mode is selected to set the four shift events, the piezoelectric driver 61 drives the shift mechanism 38 of the displacing device 32 to displace the distal tip 48 of the fiber optic image guide 31 as illustrated in Fig. 8. At first, the shift mechanism 38 displaces the distal tip 48 laterally from the initial position of (a) leftwards and downwards with an inclination of 30 degrees, and with an amount half as much as the pitch P of arrangement of the optical fibers 52. The shift mechanism 38 sets the distal tip 48 in a set position of (b) with a first shift event. Then the distal tip 48 is displaced at the same amount in the rightward and downward direction, and set in a set position of (c) with a second shift event. In sequence, the distal tip 48 is displaced at the same amount in the rightward and upward direction, and set in a set position of (d) with a third shift event. Then the distal tip 48 is displaced at the same amount in the leftward and upward direction, and set in an initial position of (a) with a fourth shift event by way of the initial position. The shift mechanism 38 is stopped in the set positions stepwise by the piezoelectric driver 61. Note that the solid line in the drawing indicates an actual position of the core 50 at the distal tip 48. The broken line indicates a previous position of the core 50 before the actual position.

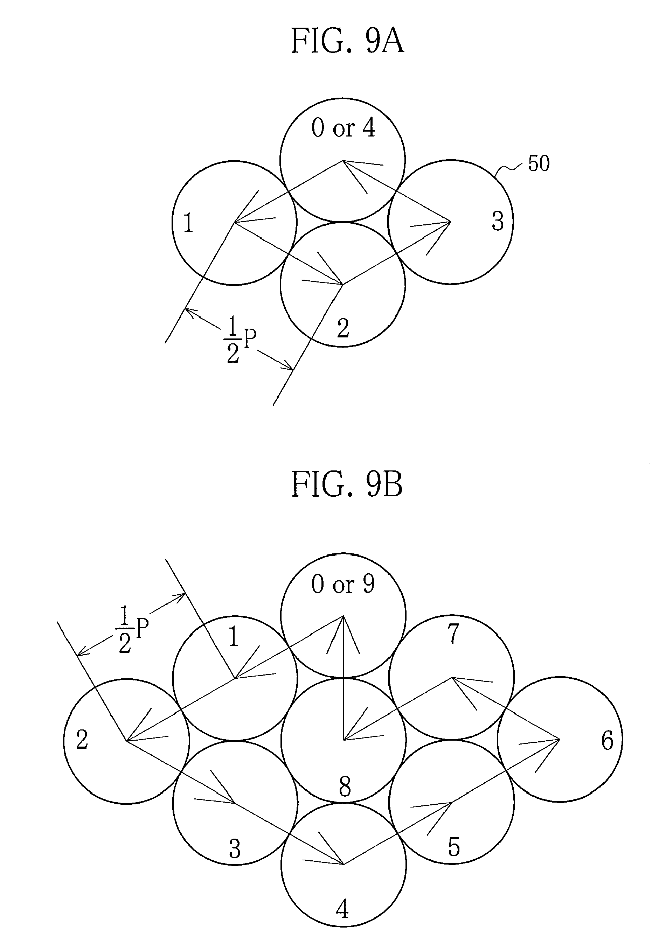

The core 50 in the distal tip 48 of the fiber optic image guide 31 repeatedly displaces in a composite sequence from the initial position of (a) to the set positions of (b), (c) and (d) then to the initial position of (a). The distal tip 48 shifts in a two-dimensional path of a polygonal shape of a rhombus of Fig. 9A to compensate for a loss region due to the cladding 51 in transmitting image light according to the initial position of (a).

Let a number of shift events be nine (9). In Fig. 9B, a two-dimensional path according to the nine shift events is illustrated. The number of shift events in each of the directions is one larger than that according to the mode of the four shift events. Note that a lateral direction from the seventh set position to the eighth set position is downward in contrast with the upward direction from the sixth set position to the seventh set position. A lateral direction from the eighth set position to the initial position or the ninth set position is upward with an angle of 90 degrees. In a manner similar to the mode of the four shift events, the two-dimensional path according to the nine shift events is in a shape to compensate for a loss region of the cladding 51 in transmitting image light according to the initial position. Furthermore, the distal tip 48 is displaced to the positions of the second, fourth and sixth set positions, which are the same as initial positions of three adjacent cores among the cores 50.

In Fig. 10, the composite imaging mode is designated. A sync control unit 62a and a piezoelectric control unit 62b are started in the CPU 62 of the processing apparatus 11. According to displacement information 85, the image synthesizing unit 65a of the digital signal processor 65 cooperates with the sync control unit 62a and the piezoelectric control unit 62b to perform various tasks.

The displacement information 85 is data related to shift events of the shift mechanism 38 of the displacing device 32. Examples of the data are a number of shift events, shift direction, shift pitch, relative positions of the pixels 81 of the CCD group 58 and the part image 80 transmitted through the core 50 of the fiber optic image guide 31 of Fig. 7. The data of the number of shift events is generated by the input interface 68. The ROM 63 stores basic datasets of the shift direction, shift pitch, relative positions of the pixels 81 and the part image 80. Any of those is read from the ROM 63 to the image synthesizing unit 65a, the sync control unit 62a and the piezoelectric control unit 62b.

The sync control unit 62a receives information of the drive pulses for the CCD group 58 from the CCD driver 60, and sends the piezoelectric control signal Sa to the piezoelectric control unit 62b and the image synthesis signal Sb to the image synthesizing unit 65a. The piezoelectric control unit 62b controls the piezoelectric driver 61 for fine displacement in synchronism with the piezoelectric control signal Sa. Similarly, the image synthesizing unit 65a performs a task of image synthesis in synchronism with the image synthesis signal Sb. Pixels of images G0, G1, G2 and G3 obtained from the set positions (in the mode of the four shift events) are mapped in accordance with the set positions, to create one synthesized image Gc.

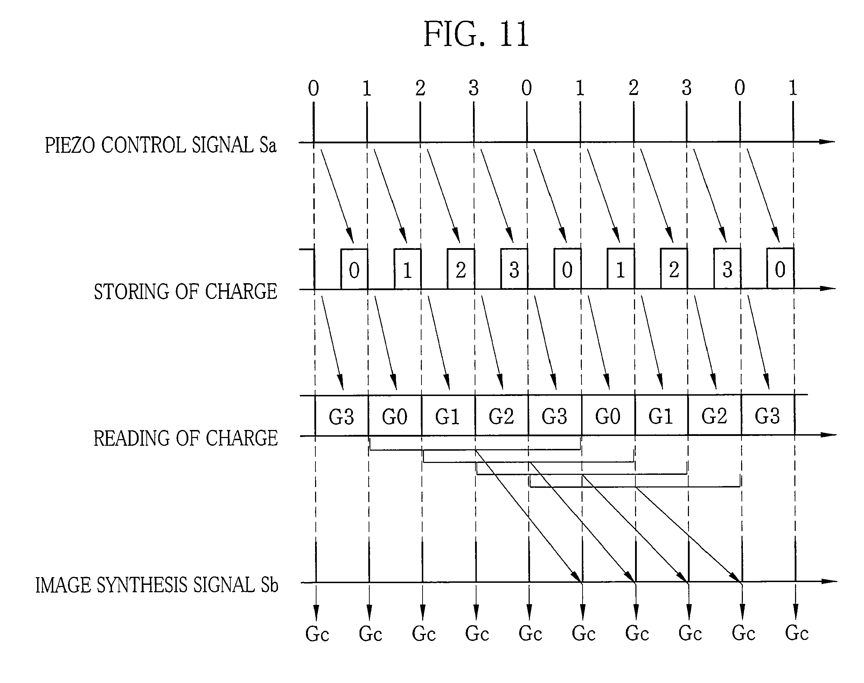

In Fig. 11, the mode of the four shift events is illustrated. Immediately after completing storing of the charge in the CCD group 58, the sync control unit 62a generates a piezoelectric control signal Sa. This is when the signal charge of one frame is read to a vertical transfer path from the pixels 81 of the CCD group 58 (or when a reading pulse is output by the CCD driver 60 to the CCD group 58). Also, the sync control unit 62a generates an image synthesis signal Sb in the same sequence as the piezoelectric control signal Sa. The operation of reading the charge is a sequence of CCD operation inclusive of reading the signal charge from the pixels 81 of the CCD group 58 to the vertical path, and vertical transfer, horizontal transfer and an output of an image signal of one frame.

The piezoelectric driver 61 in response to the piezoelectric control signal Sa supplies the piezoelectric actuator material 35 with a predetermined voltage, to displace the shift mechanism 38 from a previous set position to a present set position. Shift time from an output of the piezoelectric control signal Sa from the sync control unit 62a to the piezoelectric driver 61 until shift of the shift mechanism 38 to a succeeding set position is shorter than clearing time from completion of previous storing of charge in the CCD group 58 until a start of succeeding storing of charge. Thus, succeeding storing of charge is always started while the shift mechanism 38 is kept set in the succeeding set position by the piezoelectric driver 61.

The image synthesizing unit 65a in response to the image synthesis signal Sb reads images G0-G3 obtained from the set positions of displacement. The image synthesizing unit 65a maps pixels of the images G0-G3 according to the set positions of displacement, and outputs a synthesized image Gc. Specifically, at first, one synthesized image Gc is produced from the four images G0-G3 obtained in a two-dimensional shift sequence. Then another synthesized image Gc is created by reuse of the three images G1-G3 upon creation of the previous synthesized image Gc and use of a new image G0 obtained in a succeeding two-dimensional shift sequence. Similarly, a synthesized image Gc is created by reuse of the three images G2, G3 and G0 upon creation of the previous synthesized image Gc and use of a new image G1 obtained in response to the image synthesis signal Sb. A further succeeding synthesized image Gc is created by reuse of the three images G3, G0 and G1 upon creation of the previous synthesized image Gc and use of a new image G2 obtained in response to the image synthesis signal Sb. In short, the image synthesizing unit 65a creates the synthesized image Gc consecutively without interruption between shift events by changing combinations of four consecutive images among numerous images G0, G1, G2, G3, G0, G1 and so on obtained sequentially. The composite imaging mode of the nine shift events is similar. Image synthesis is carried out to produce a synthesized image Gc consecutively without interruption from the nine images obtained according to the set positions of fine displacement and by additionally using a new image of one set position in place of the oldest one of images in a previous combination. For this construction, a specific frame memory is incorporated in the digital signal processor 65 for set positions. The image synthesizing unit 65a selectively reads combinations of images required for creating the synthesized image Gc. Also, it is possible to prepare a plurality of frame memories as many as the set positions, and to update a memory location of the oldest image by a newly obtained image in a queue method of updating in which oldest data is deleted and dequeued upon input of new data with priority. It is further possible to interpolate the pixels to process the images G0-G3 or synthesized image Gc in the course of the synthesis.

In the synthesized image Gc, a loss region due to the cladding 51 in transmitting image light can be compensated for in a visible manner. Pixel values of the pixels of the portions are directly derived from the object image without approximation or interpolation of adjacent pixels within one frame. Consequently, the number of pixels is higher than that in images obtained from the normal imaging mode or according to each one of the set positions of displacement, to produce the image in a very fine quality. Note that the image quality is higher in images obtained according to the nine shift events than images of the four shift events.

It is to be noted that the images G0-G3 are different part images 80 with differences in the set position by displacement. The part image 80 at the distal tip 48 is only shifted by keeping a proximal tip of the fiber optic image guide 31 stationary. No change occurs in the relative position between the proximal tip of the fiber optic image guide 31 and an image pickup surface of the CCD group 58. There are no apparently distinct features between data output according to the pixels 81 even with the various set positions. For example, the part image 80 of a position in the image G0 is different from the part image 80 of the same position in the image G1 in relation to the set position. However, those are recorded commonly by the pixels 81 on the CCD group 58. Accordingly, the image synthesizing unit 65a determines original pixels of pixel values of the images among the pixels 81 by mapping on the basis of the relative position of the part image 80 of the displacement information 85 and the pixels 81, to carry out the pixel interpolation.

The operation of the endoscope system 2 of the above construction is described now. To observe a body part of a patient endoscopically, a doctor or operator connects the endoscope 10 to the processing apparatus 11 and the light source apparatus 12, which are turned on and powered. The input interface 68 is manually operated to input information related to the patient, to start examination.

After instructing the start, the doctor or operator enters the elongated tube 13 in the body. Light from the light source apparatus 12 is applied to body parts, while he or she observes an image on the display panel 21 from the CCD group 58 of the image sensor.

An image signal is generated by the CCD group 58, processed by the analog front end 59 for various functions of processing, and input to the digital signal processor 65. The digital signal processor 65 processes the image signal for various functions of signal processing, to produce an image. The image from the digital signal processor 65 is output to the digital image processor 66.

The digital image processor 66 is controlled by the CPU 62 and processes the image from the digital signal processor 65 for various functions of image processing. The image is input by the digital image processor 66 to the display control unit 67. According to the graphic data from the CPU 62, the display control unit 67 carries out control for display. Thus, the image is displayed on the display panel 21 as an endoscopic image.

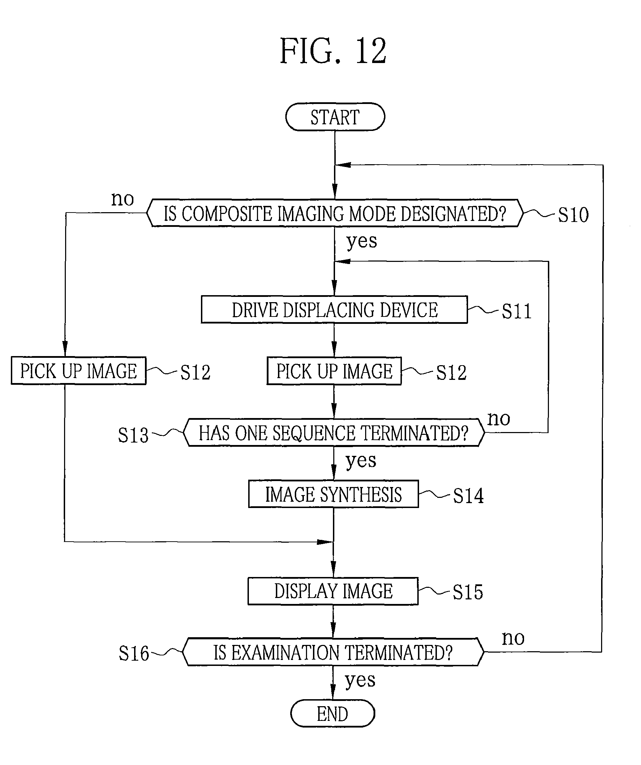

In Fig. 12, a composite imaging mode is designated (yes at the step S10). The sync control unit 62a and the piezoelectric control unit 62b are ready in the CPU 62 in the processing apparatus 11. According to the displacement information 85 and information of drive pulses from the CCD driver 60 for the CCD group 58, the sync control unit 62a sends the piezoelectric control signal Sa to the piezoelectric control unit 62b, and sends the image synthesis signal Sb to the image synthesizing unit 65a.

The operation of the piezoelectric driver 61 is controlled by the piezoelectric control unit 62b upon receiving the piezoelectric control signal Sa. The piezoelectric driver 61 applies a predetermined voltage to the piezoelectric actuator material 35. Thus, the shift mechanism 38 displaces at a predetermined angle and pitch according to the designated number of the shift events. See the step S11. At each time that the shift mechanism 38 is retained in one of the set positions, charge is stored in the CCD group 58. The part image 80 of a body part is picked up by the pixels 81 through the fiber optic image guide 31 in the step S12. A sequence including the steps S11 and S12 is repeated (no at the step S13) until the end of one two-dimensional shift sequence by shift of the shift mechanism 38 from the initial position and again to the same position.

When one two-dimensional shift sequence is terminated (yes at the step S13), image synthesis is carried out by the image synthesizing unit 65a upon receiving the image synthesis signal Sb, to produce a synthesized image at the step S14 consecutively without interruption from the images obtained according to the set positions of fine displacement. After data of the synthesized image is processed in the digital image processor 66 and the display control unit 67, the synthesized image is displayed on the display panel 21 at the step S15. In contrast, when the normal imaging mode is designated, an image is picked up in the step S12 but without carrying out the sequence of the steps S11 and S14. Those steps are repeated until a command signal for terminating the examination is input (yes at the step S16).

As described above, the distal tip 48 of the fiber optic image guide 31 is displaced by the piezoelectric actuator material 35, to record plural images in one two-dimensional shift sequence. One synthesized image is obtained by combining the plural images. Thus, the image of high quality for diagnosis can be obtained, and the elongated tube 13 can have a very small diameter as ultra thin tube. In the long series of the images G0-G3 created successively, combinations of four images are changed by one image. An oldest one of the four images of each combination is replaced by a newly created image successively, so that a synthesized image Gc is created without interruption for each one of shift events. Thus, there is no drop in the frame rate. A moving image of smooth movement suitable for diagnosis can be provided.

An outer diameter of the displacing device 32 containing the fiber optic image guide 31 is equal to or lower than that of the lens barrel 33 inclusive of the connection cable 45. A thickness of each constituent of the displacing device 32 is as small as tens of microns. There occurs no increase of the size of the displacing device 32 in the radial direction. It is possible to reduce the extremely small diameter by extreme thinning in comparison with the known techniques for displacing the lens system for imaging.

The sync control unit 62a synchronizes the displacing device 32 with the CCD group 58. Images are picked up in setting the fiber optic image guide 31 in each one of the set positions. Thus, clear images can be recorded without blur in the set positions, to create a synthesized image with high quality.

The coating of the piezoelectric actuator material 35 is applied to the support casing 34 having a cylindrical shape. Voltage is applied to the two pairs of the electrodes 36. Thus, the shift mechanism 38 displaces in Figs. 9A and 9B in the shortest two-dimensional path with the set positions in a patterned loop of a certain polygon. The shift mechanism 38 can shift to follow the image pickup of the CCD group 58 with a quick response without taking much time for fine displacement.

In the shift mechanism 38, the electrodes 36 have the large width to exert great force for driving. The narrow portion 43 of the electrodes 36 in the stationary section 39 is effective in preventing exertion of force to the stationary section 39. This results in high efficiency in driving and maintenance of high mechanical strength.

As the connection cable 45 is connected to the electrodes 36 on a proximal side from the stationary section 39, no mechanical stress is exerted to the connection cable 45 even upon the displacement. An increase of the size in the radial direction can be small in the structure of disposing the connection cable 45 about the support casing 34.

The fiber optic image guide 31 is held by the support casing 34 where the piezoelectric actuator material 35 is present. This makes it easy to assemble parts of the structure in comparison with a structure of the piezoelectric actuator material 35 directly on the fiber optic image guide 31. Also, the support casing 34 is used as a common electrode of the piezoelectric actuator material 35, so as to reduce the number of electrodes and cables. This is effective in reducing the diameter of the endoscope with an ultra thin tube.

It is possible selectively to set the normal imaging mode and the composite imaging mode, so as to reflect the intention of the doctor or operator. When the elongated tube 13 of the endoscope 10 is entered in the body and advanced, the normal imaging mode is sufficient, because a motion image can be created smoothly in a continuous manner without delay of time relative to motion of an object even with a low image quality. In contrast with this, the composite imaging mode is effectively designated for detailed imaging after the reach of the distal end of the elongated tube 13 to an object of interest with affected tissue or the like, so that an image suitable for diagnosis can be provided because of its higher image quality than the normal imaging mode.

Also, it is possible automatically to change over to a composite imaging mode of a high quality when the recording button 18 is depressed for recording a still image. For example, the composite imaging mode of the nine shift events is set upon depression of the recording button 18 in the course of a normal imaging mode or composite imaging mode of the four shift events. Thus, a still image of a body part can be endoscopically recorded with high quality, and can be utilized for diagnosis.

As the light guide devices 27 or illumination fiber optics are randomly packed in the remaining tube lumen in the elongated tube 13 as viewed in the distal end face 20a, light can be dispersed in a large area. The three-CCD assembly 56 is used, to produce a highly precise image, as the number of pixels is large in comparison with a single CCD assembly.

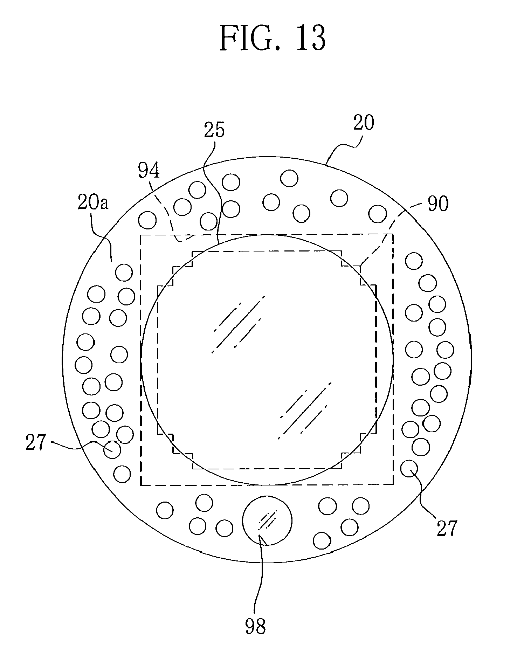

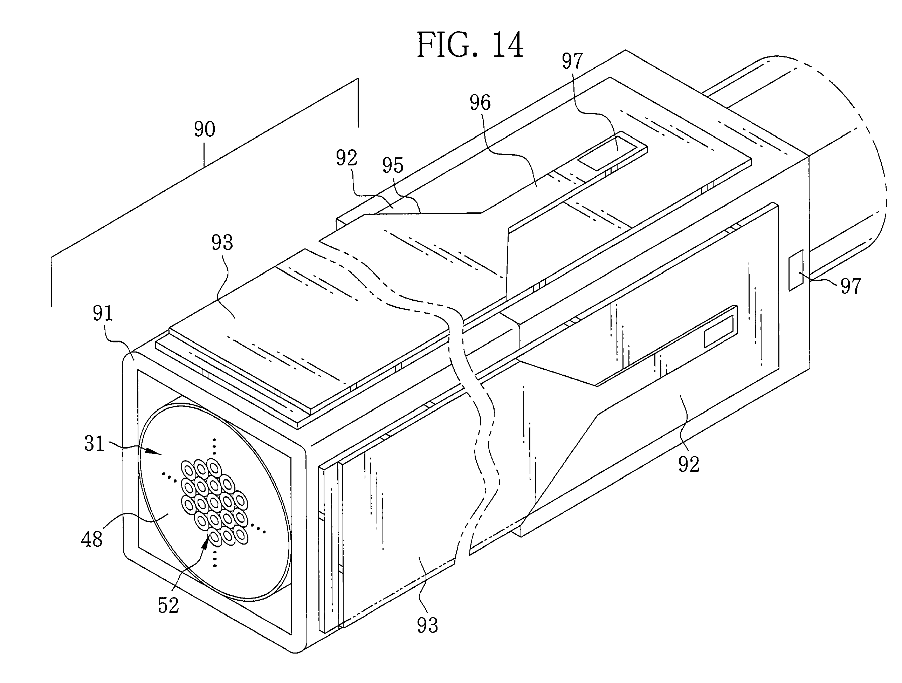

A displacing device may be shaped in a form other than the rod form. In Figs. 13 and 14, another preferred displacing device 90 of a shape of a quadrilateral prism is illustrated. Elements similar to the above embodiments are designated with identical reference numerals.

A support casing 91 is included in the displacing device 90 in a form of a quadrilateral prism. The support casing 91 is constituted by a tube of a stainless steel and has a thickness of 50 microns and a width of 0.7 mm. The fiber optic image guide 31 is inserted in and attached to the support casing 91 by an adhesive agent (not shown) or the like. A piezoelectric actuator material 92 has a thickness of 50 microns, and is a coating overlaid on four faces of the support casing 91, or is a film or sheet attached to the four faces of the support casing 91 with electrically conductive adhesive agent. Electrodes 93 are a coating about the piezoelectric actuator material 92.

The displacing device 90 is contained in the wall of the head assembly 20. A lumen 94 is defined between the outside of the displacing device 90 and the inside of the wall of the head assembly 20, and has a width of approximately 0.1 mm.

The electrodes 93 are two pairs in the same manner as the above embodiment. A narrow portion 96 is defined as a portion of the electrodes 93 between recesses 95. A pad 97 is disposed at an end of the narrow portion 96 and the support casing 91, and used for connection with the connection cable 45.

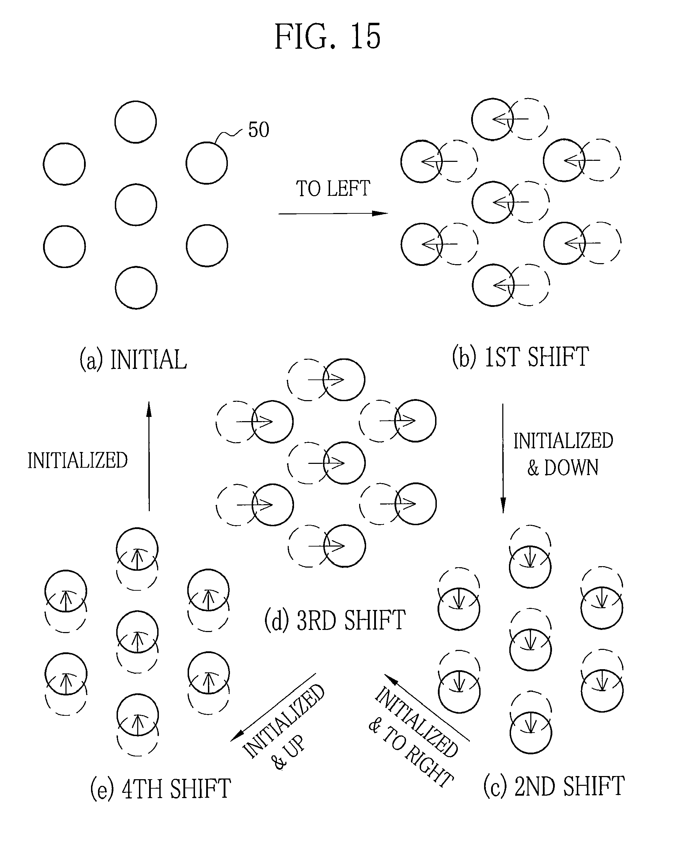

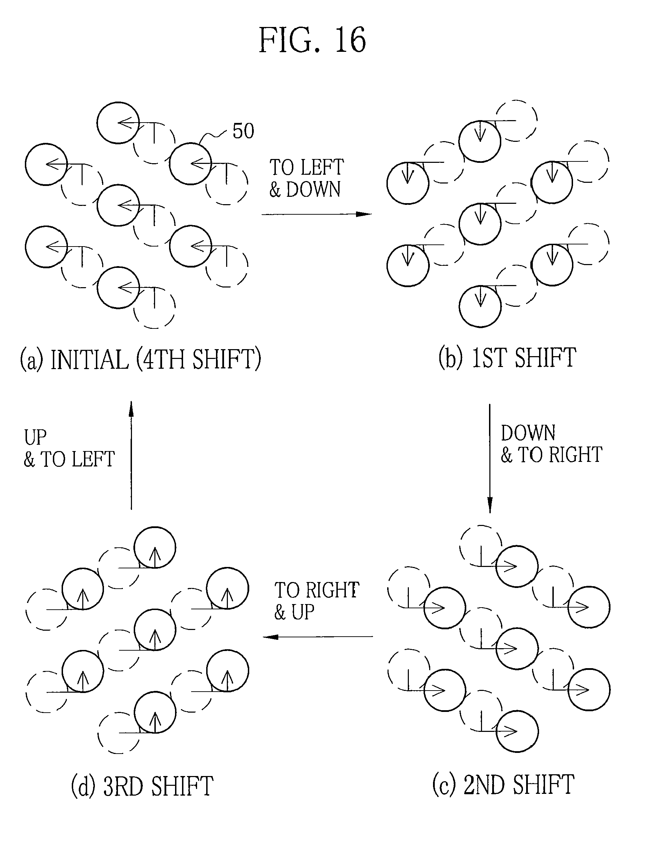

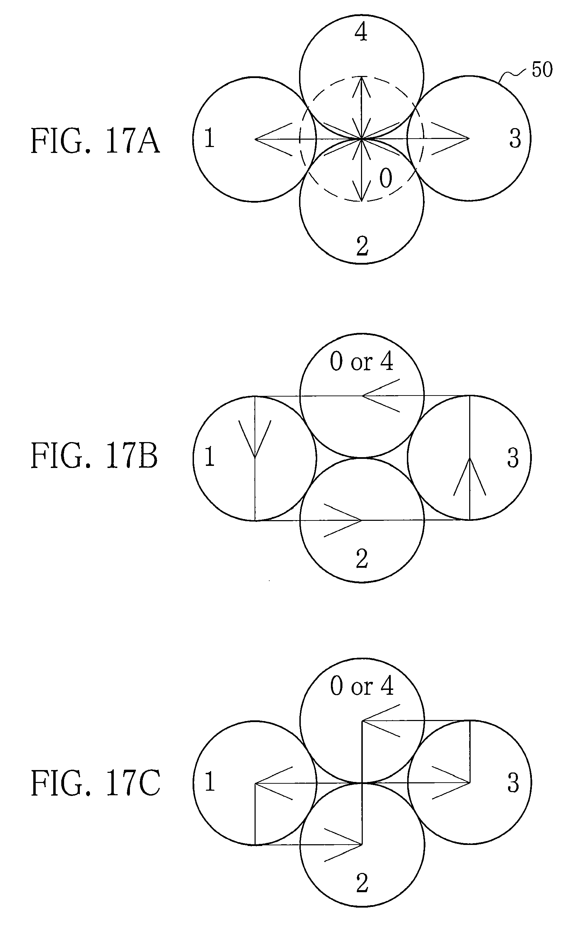

The shift mechanism 38 displaces in the manner of Fig. 15 or 16 in the displacing device 90. In Fig. 15, the shift mechanism 38 displaces from the initial position of (a) in the leftward direction by 90 degrees at an amount of $\sqrt{3}/4 \cdot P$ to come to the set position of (b) with a first shift event. After the image pickup in the set position of (b) with the first shift event, the shift mechanism 38 is returned to the initial position, and displaces in the downward direction by 90 degrees at an amount of 1/4.P to come to the set position of (c) with a second shift event. Then the shift mechanism 38 is returned to the initial position, and displaces in the rightward direction by 90 degrees at an amount of $\sqrt{3}/4 \cdot P$ to come to the set position of (d) with a third shift event. The shift mechanism 38 is returned to the initial position, and displaces in the upward direction by 90 degrees at an amount of 1/4.P to come to the set position of (e) with a fourth shift event. Finally, the shift mechanism 38 is returned to the initial position. The core 50 at the distal tip 48 of the fiber optic image guide 31 displaces in the two-dimensional path of a crossed shape or polygonal chain of Fig. 17A by fine displacement to the set positions (b), (c), (d) and (e) and return to the initial position.

Also, in Fig. 16, the shift mechanism 38 displaces from the initial position of (a) in the leftward direction by 90 degrees at an amount of $\sqrt{3}/4 \cdot P$, and displaces in the downward direction by 90 degrees at an amount of 1/4.P, to come to the set position of (b) with a first shift event. After the image pickup in the set position of (b) with the first shift event, the shift mechanism 38 displaces from the set position of (b) in the downward direction by 90 degrees at an amount of 1/4.P, and displaces in the rightward direction at an amount of $\sqrt{3}/4 \cdot P$, to come to the set position of (c) with a second shift event. Also, in Fig. 16, the shift mechanism 38 displaces from the set position of (c) in the rightward direction by 90 degrees at an amount of $\sqrt{3}/4 \cdot P$, and displaces in the upward direction by 90 degrees at an amount of 1/4.P, to come to the set position of (d) with a third shift event. After the image pickup in the set position of (d) with the third shift event, the shift mechanism 38 displaces from the set position of (d) in the upward direction by 90 degrees at an amount of 1/4.P, and displaces in the leftward direction at an amount of $\sqrt{3}/4 \cdot P$. Finally, the shift mechanism 38 is returned to the initial position of (a). The core 50 at the distal tip 48 of the fiber optic image guide 31 displaces in the two-dimensional path of a quadrilateral shape of Fig. 17B by repetition of return to the initial position (a) and displacing to the set positions (b), (c) and (d).

It is also possible to displace the distal tip 48 in a two-dimensional shift sequence of Fig. 17C, namely a two-dimensional path of a form of a crooked cross or gourd shape, or in the downward and leftward direction, the downward and rightward direction, the upward and rightward direction, and the upward and leftward direction. An upward or downward shift is carried out at first. In a manner similar to the above embodiment, the distal tip 48 is displaced to compensate for a loss region of the cladding 51 in transmitting image light according to the initial position.

In the above embodiments, the piezoelectric actuator material 35 is overlaid in a cylindrical form on the displacing device 32, which can have the outer diameter equal to that of the lens barrel 33. It is possible to reduce the diameter of the elongated tube 13 more effectively for an ultra thin tube. In Figs. 13 and 14, corners of the displacing device 90 having a shape of the quadrilateral prism project from the profile of the lens barrel 33. It is easy to dispose the piezoelectric actuator material 92 on the displacing device 90 by coating or adhesion without difficulty in manufacture. The displacing device 90 can be produced inexpensively and easily although a diameter of the elongated tube 13 is larger than that of the displacing device 32.

It is possible to utilize the displacing device 32 with the example of Figs. 15-17C in which displacement is carried out with angular differences of 90 degrees. In Fig. 13, there is a lighting window 98 where a distal end face is extended forwards for applying laser light for treatment to affected tissue as object of interest. The lighting window 98 is an alternative structure in place of the working channel 46. Also, the lighting window 98 may be incorporated with the displacing device 32.

The above-described displacing device and method are only examples. Variants of displacing methods are possible. For example, the third set position may be omitted from the four set positions of fine displacement. It is possible to actuate the displacing device for three shift events in directions of 30 degrees, and return the same to the initial position. In short, the two-dimensional path can be triangular. Also, the eighth set position may be omitted from the nine set positions of displacement. It is possible to actuate the displacing device for eight shift events in directions of 30 degrees, and return the same to the initial position. Furthermore, the displacing device can displace to the first and second set positions included in the nine set positions, and return to the initial position.

Examples of two-dimensional paths where set positions are arranged according to the invention include a polygon, a concave polygon, one polygonal path of an open form, and plural polygonal paths which share one or more common point.

However, there is a characteristic of the hysteresis in the piezoelectric actuator materials, of which the set position may be offset upon driving the piezoelectric actuator materials without a patterned manner. Thus, the displacing device is caused to displace with the same two-dimensional path and in the same sequence. In short, a sequence of driving the piezoelectric actuator materials for actuating the displacing device is set equal for every event. Also, a sequence of supplying electrodes with voltage on a couple of the right and left sides is kept the same.

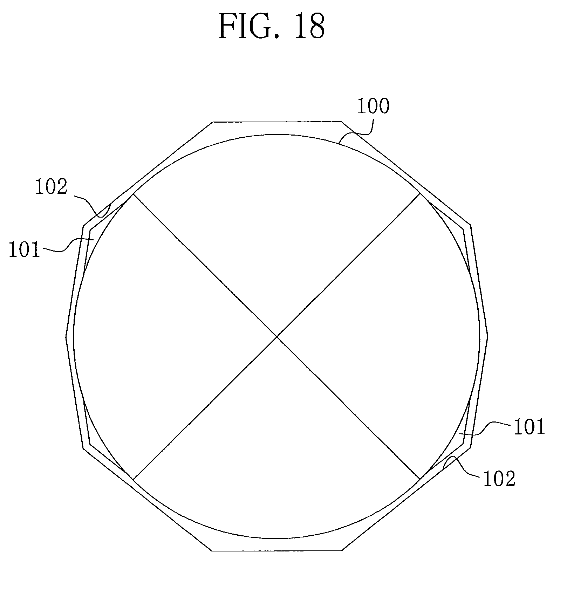

In the above embodiment, the displacement of the fiber optic image guide 31 is electrically controlled by changing the voltage applied to the piezoelectric actuator material 35. In addition to this or instead of this, displacement of the displacing device 32 in the set positions may be controlled mechanically. In Fig. 18, a displacing device 100 is illustrated, and displaces in the same manner as the displacing device 32. Regulating projections 101 are formed on the displacing device 100 and positioned in the shift direction of the outer surface of the distal end. Regulating recesses 102 are formed on a wall of the head assembly 20 for containing the displacing device 100, and disposed to face the projections 101. The recesses 102 are distant from the projections 101 with a lumen suitable for displacement of the displacing device 100. When the displacing device 100 displaces, the projections 101 become received in the recesses 102 to retain the displacing device 100.

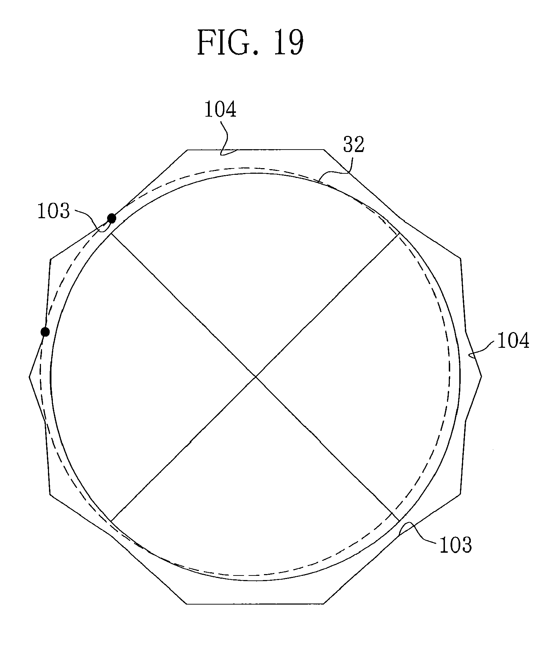

In Fig. 19, regulating projections 103 are formed on an inner wall of the head assembly 20. When the displacing device 32 displaces to the set positions, an outer surface of the displacing device 32 contacts the projections 103 at two points, which are indicated by dots for clarity in the drawing. There are escapement recesses 104 for allowing the displacing device 32 to displace to the set positions. As a result, a cost for manufacturing this structure can be smaller than that of Fig. 18, because no projection is required for the displacing device 32.

To displace the distal tip 48 of the fiber optic image guide, only a portion of the fiber optic image guide in front of the stationary section is displaced unlike a structure in which a lens or optics for focusing are shifted. Force applied to the fiber optic image guide by displacement includes a component applied by the piezoelectric actuator material and a component of reaction force to come back to its original position. As the weight of the fiber optic image guide is comparatively great, it is likely that the fiber optic image guide does not displace smoothly due to the reaction force. However, a structure of Fig. 18 mechanically retains the fiber optic image guide in a set position. Thus, the fiber optic image guide can have the set position without error, and can operate stably and rapidly for displacement. Relatively low precision for control of the voltage can be sufficient with advantage.

The head assembly is entered in the body, and cleaned, disinfected or sterilized after use. The head assembly is likely to stand in the environment of high humidity. Thus, it is preferable to apply a moisture-proof coating to a displacing device inclusive of the cable, before the displacing device can be mounted in the head assembly. An example of the coating is a parylene coating which can be applied at a uniform thickness by the chemical vapor deposition (CVD) of low vacuum and low temperature.

As the fiber optic image guide is displaced by tilt of a root portion of the shift mechanism, the fiber optic image guide is likely to vibrate and stop with lag in the set positions without immediate stop. Thus, it is preferable with a piezoelectric driver to drive the piezoelectric actuator material or to use other anti-vibration methods in order to tilt the shift mechanism instantaneously in reverse to the displacing after a stop of the displacing device. Specifically, reaction force is previously measured by simulation or experimentally. An offset voltage for the piezoelectric actuator material is stored in a ROM. The piezoelectric control unit reads the information of the offset voltage from the ROM and sets the information in the piezoelectric driver. Furthermore, non-conductive fluid with high viscosity can be charged in a lumen for an anti-vibration structure by utilizing damping effect.

In the above embodiment, shift time for the shift mechanism to displace to a succeeding set position is shorter than clearing time from previous completion of storing charge of the CCD until a succeeding start of storing charge. However, the shift time may be longer than the clearing time for the reason of various factors, which include a length, material or shift amount of the shift mechanism, performance of the piezoelectric actuator material, or the like. In considering that the weight of the fiber optic image guide is relatively large, the shift time is very likely to be longer than the clearing time.

While the shift mechanism is set in the set position, the CCD driver is controlled by the CPU of the processing apparatus and supplies the CCD with an electronic shutter pulse. A start of storing charge is delayed. When the shift mechanism stops in the set position, storing charge is started. Otherwise, the light source is turned off while the shift mechanism is set in the set position, and then turned on when the shift mechanism stops in the set position.

In order to drive the CCD with reference to time required for shift to a succeeding one of the set positions, the frame rate must be set small should the shift time be longer than the clearing time. However, it is possible without blur to obtain an image even with the presently high frame rate by sweeping out charge with an electronic shutter pulse, or by turning off the light source, or by other methods.

In the above embodiment, the image synthesizing unit synthesizes the image only when the composite imaging mode is designated. However, it is possible to synthesize an image also in the normal imaging mode. This is effective in compensating for a loss region of the cladding even though an image for reflecting an object image positioned in association with the cladding cannot be obtained. Image quality can be high.

Elements of the hardware can be incorporated in a housing separate from the processing apparatus, or may be incorporated in the endoscope, the hardware including the three-CCD assembly, the input interface for setting the imaging mode and the number of shift events, and electronic circuits to constitute the image synthesizing unit, sync control unit and piezoelectric control unit.

In Fig. 22, one preferred light source apparatus 121 for an endoscope system 120 is illustrated. The light source apparatus 121 includes a blue laser light source 122, a collimator lens 123 and a condenser lens 124. The blue laser light source 122 has a characteristic with a central wavelength of 445 nm. The collimator lens 123 collimates laser light from the blue laser light source 122 to output parallel light. The condenser lens 124 condenses the laser light. The CPU 74 controls the light source driver 71 to drive the blue laser light source 122.

Laser light emitted by the blue laser light source 122 is condensed by the condenser lens 124 and becomes incident upon a proximal end of the light guide devices 27. The light guide devices 27 transmit the laser light to the head assembly 20 of the endoscope 10.

A wavelength conversion device 125 is disposed on an exit side of the light guide devices 27 at its distal end. The wavelength conversion device 125 is a single block of a composite material which contains plural phosphors dispersed together. The phosphors partially absorb the laser light from the blue laser light source 122, to emit converted light with colors from green to yellow by excitation. The laser light from the blue laser light source 122 and the excitation light from green to yellow after the conversion are coupled together, to produce white light.

It is possible to obtain sufficiently bright light with a small number of light guide devices, namely one or two, because white light can be delivered by the blue laser light source 122 and the wavelength conversion device 125 with higher brightness than the former embodiment. The diameter of the endoscope can be reduced more effectively for an ultra thin tube.

Note that a single CCD assembly may be used instead of a three-CCD assembly. In the above embodiments, the first connector 15 is used commonly for the connection of the fiber optic image guide and the connection cable to the processing apparatus. However, two separate connectors may be used and may contain the fiber optic image guide and the connection cable discretely.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

**Claims**

1. An endoscope system comprising:

   an objective lens system (30), disposed in an elongated tube (13) of an endoscope (10), for passing image light from an object (80);
   a fiber optic image guide (31), including plural optical fibers (52) bundled together, having a distal tip (48), inserted through said elongated tube, for transmitting said image light focused on said distal tip by said objective lens system in a proximal direction;
   an image sensor (56) for detecting said image light from said fiber optic image guide;
   a displacing device (32, 90, 100) for displacing said distal tip laterally and periodically upon receiving entry of said image light being focused by use of a piezoelectric actuator (35, 92) positioned outside said distal tip;
   a sync control unit (62a) for driving said image sensor for detecting said image light for plural times in synchronism with displacement of said displacing device, and controlling said image sensor and said displacing device to create plural images in plural set positions of said distal tip relative to said image light being focused;
   an image synthesizing unit (65a) for combining first to Nth images being N of said plural images created consecutively to form a first synthesized image, and then for combining second to (N+1)th images being N of said plural images without use of said first image to form a second synthesized image, to output plural synthesized images consecutively by repeating forming of said first and second synthesized images, where N is an integer of two or more.

2. An endoscope system as defined in claim 1, wherein said displacing device shifts said distal tip stepwise from a first set position to an Nth set position, and shifts back said distal tip to said first set position for one two-dimensional shift sequence;
   said image sensor carries out image pickup for each of said first to Nth set positions.

3. An endoscope system as defined in claim 2, wherein said displacing device retains said distal tip in each of said first to Nth set positions by intermittent shifting.

4. An endoscope system as defined in claim 3, wherein a distance between said first to Nth set positions is 1/n as long as a pitch of arrangement of said optical fibers in said fiber optic image guide, wherein n is a positive integer.

5. An endoscope system as defined in claim 3, wherein N is 4 or 9, and said first to Nth set positions are arranged in a rhombic shape in which two or three of said set positions are arranged on one edge thereof and which has interior angles of substantially 60 and 120 degrees.

**6.** An endoscope system as defined in claim 2, wherein said displacing device shifts said distal tip with a shortest path length defined by arranging said first to Nth set positions.

**7.** An endoscope system as defined in claim 2, wherein said first to Nth set positions are arranged on a polygonal path.

**8.** An endoscope system as defined in claim 1, wherein said sync control unit controls a plurality of said piezoelectric actuator in a predetermined sequence.

**9.** An endoscope system as defined in claim 1, further comprising:

> a laser light source for supplying said light guide devices with laser light; and
> a wavelength conversion device, disposed at a distal end of said light guide devices, for emitting light by excitation with said laser light, to obtain white light by mixing said excitation light with said laser light.

**10.** An endoscope system as defined in claim 1, wherein each of said optical fibers includes:

> a core; and
> a cladding disposed about said core;
> said displacing device shifts said distal tip at a shift amount for setting a distal end of said core at a location where a distal end of said cladding has been set.

**11.** An endoscope comprising:

> an elongated tube (13);
> an objective lens system (30), disposed in said elongated tube, for passing image light from an object (80);
> a fiber optic image guide (31), including plural optical fibers (52) bundled together, having a distal tip (48), inserted through said elongated tube, for transmitting said image light focused on said distal tip by said objective lens system in a proximal direction;
> a displacing device (32, 90, 100) for displacing said distal tip laterally and periodically upon receiving entry of said image light being focused by use of a piezoelectric actuator (35, 92) positioned outside said distal tip;
> a support casing (34, 91) for supporting said distal tip inserted therein, keeping said distal tip shiftable on said displacing device, and transmitting force of said piezoelectric actuator disposed outside to said fiber optic image guide;
> wherein said fiber optic image guide transmits said image light to an image sensor (56) for detecting said image light for plural times in synchronism with displacement of said displacing device, said image sensor and said displacing device are controlled to create plural images in plural set positions of said distal tip relative to said image light being focused;
wherein first to Nth images being N of said plural images created consecutively are combined to form a first synthesized image, and then second to (N+1)th images being N of said plural images are combined without use of said first image to form a second synthesized image, to output plural synthesized images consecutively by repeating forming of said first and second synthesized images, where N is an integer of two or more.

**12.** An endoscope as defined in claim 11, wherein said displacing device includes:

> a stationary section secured inside said elongated tube;
> a shift mechanism, disposed to extend from said stationary section in a distal direction, for displacing said distal tip.

**13.** An endoscope as defined in claim 12, wherein said piezoelectric actuator extends fully along said displacing device inclusive of said stationary section and said shift mechanism;
an electrode of said piezoelectric actuator extends to a proximal end of said stationary section.

**14.** An endoscope as defined in claim 12, further comprising a regulating portion, formed with any one of said displacing device and an inner surface of said elongated tube, for regulating an orientation of said displacing device.

**15.** A driving method of driving an endoscope (10) including an elongated tube (13), an objective lens system (30), disposed in said elongated tube, for passing image light from an object (80), and a fiber optic image guide (31), including plural optical fibers (52) bundled together, having a distal tip (48), inserted through said elongated tube, for transmitting said image light focused on said distal tip by said objective lens system in a proximal direction, said driving method comprising steps of:

> displacing said distal tip laterally and periodically upon receiving entry of said image light being focused by use of a piezoelectric actuator (35, 92) positioned outside said distal tip;
> driving an image sensor (56) for detecting said image light for plural times in synchronism with displacement, to create plural images with said image sensor in plural set positions of said distal tip relative to said image light being focused;

combining first to Nth images being N of said plural images created consecutively to form a first synthesized image, and then combining second to (N+1) th images being N of said plural images without use of said first image to form a second synthesized image, to output plural synthesized images consecutively by repeating forming of said first and second synthesized images, where N is an integer of two or more.

# FIG. 1

EP 2 266 453 A1

# FIG. 2

FIG. 3

# FIG. 4

EP 2 266 453 A1

# FIG. 5

FIG. 6

FIG. 7

# FIG. 8

(a) INITIAL (4TH SHIFT)

(b) 1ST SHIFT

(d) 3RD SHIFT

(c) 2ND SHIFT

# FIG. 9A

# FIG. 9B

# FIG. 10

EP 2 266 453 A1

# FIG. 11

PIEZO CONTROL SIGNAL Sa

STORING OF CHARGE

READING OF CHARGE

IMAGE SYNTHESIS SIGNAL Sb

# FIG. 12

```
                    START

         no
    ┌─────────< IS COMPOSITE IMAGING MODE DESIGNATED? >~S10
    │                              │ yes
    │                              │
    │                    ┌──────────────────────┐
    │                    │ DRIVE DISPLACING DEVICE │~S11
    │                    └──────────────────────┘
    │                              │
┌────────────┐          ┌──────────────┐
│ PICK UP IMAGE │~S12    │ PICK UP IMAGE │~S12
└────────────┘          └──────────────┘
    │                              │
    │      S13~< HAS ONE SEQUENCE TERMINATED? >──no
    │                              │ yes
    │                    ┌──────────────┐
    │                    │ IMAGE SYNTHESIS │~S14
    │                    └──────────────┘
    │                              │
    └──────────────────────────────┤
                         ┌──────────────┐
                         │ DISPLAY IMAGE │~S15
                         └──────────────┘
                                    │
              S16~< IS EXAMINATION TERMINATED? >──no
                                    │ yes
                                  END
```

# FIG. 13

FIG. 14

EP 2 266 453 A1

# FIG. 15

(a) INITIAL

TO LEFT

(b) 1ST SHIFT

INITIALIZED

INITIALIZED & DOWN

(d) 3RD SHIFT

INITIALIZED & UP

INITIALIZED & TO RIGHT

(e) 4TH SHIFT

(c) 2ND SHIFT

# FIG. 16

(a) INITIAL (4TH SHIFT)

(b) 1ST SHIFT

(d) 3RD SHIFT

(c) 2ND SHIFT

TO LEFT
& DOWN

DOWN
& TO RIGHT

UP
& TO LEFT

TO RIGHT
& UP

FIG. 17A

FIG. 17B

FIG. 17C

FIG. 18

# FIG. 19

# FIG. 20

EP 2 266 453 A1

Europäisches
Patentamt

European
Patent Office

Office européen
des brevets

## PARTIAL EUROPEAN SEARCH REPORT

**Application Number**

under Rule 62a and/or 63 of the European Patent Convention.
This report shall be considered, for the purposes of
subsequent proceedings, as the European search report

EP 10 16 5665

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/076772 A1 (OPTISCAN PTY LTD [AU]; HARRIS MARTIN RUSSELL [AU]) 27 July 2006 (2006-07-27) * page 1, line 10 - line 12 * * page 2, line 30 - line 34 * * page 4, line 21 - page 6, line 25 * ----- | 1-10 | INV. A61B1/005 |
| X | US 6 294 775 B1 (SEIBEL ERIC J [US] ET AL) 25 September 2001 (2001-09-25) * column 8, line 64 - column 9, line 51 * ----- | 1-10 | |
| X | US 5 103 497 A (HICKS JOHN W [US]) 7 April 1992 (1992-04-07) * column 4, line 1 - line 7 * * column 5, line 38 - line 51 * ----- -/-- | 1-10 | |

TECHNICAL FIELDS
SEARCHED      (IPC)

A61B

### INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 16 November 2010 | Marteau, Frédéric |

EPO FORM 1503 03.82 (P04E07)

Europäisches
Patentamt

European
Patent Office

Office européen
des brevets

**PARTIAL EUROPEAN SEARCH REPORT**

Application Number

EP 10 16 5665

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | US 2007/273930 A1 (BERIER FREDERIC [FR] ET AL) 29 November 2007 (2007-11-29)<br>* abstract *<br>* paragraph [0006] *<br>* paragraph [0015] *<br>* paragraph [0018] - paragraph [0025] *<br>* paragraph [0035] *<br>* paragraph [0045] - paragraph [0046] *<br>* paragraph [0060] - paragraph [0063] *<br>* paragraph [0067] - paragraph [0068] *<br>* claim 1 *<br>* figures 1-4 *<br>----- | 1-10 | |
| A | US 2009/059359 A1 (NAHM WERNER [DE] ET AL) 5 March 2009 (2009-03-05)<br>* paragraph [0014] *<br>----- | 1,9 | **TECHNICAL FIELDS SEARCHED (IPC)** |
| A | US 4 154 502 A (SIEGMUND WALTER P [US]) 15 May 1979 (1979-05-15)<br>* column 1, line 12 - line 22 *<br>----- | 10 | |

EPO FORM 1503 03.82 (P04C10)

Europäisches
Patentamt

European
Patent Office

Office européen
des brevets

**INCOMPLETE SEARCH
SHEET C**

Claim(s) completely searchable:
      1-10

Claim(s) not searched:
      11-15

Reason for the limitation of the search:

The search has been restricted to the subject-matter indicated by the
applicant in his letter of 14-10-2010 filed in reply to the invitation
pursuant to Rule 62a(1) and Rule 63(1) EPC.

## ANNEX TO THE EUROPEAN SEARCH REPORT
## ON EUROPEAN PATENT APPLICATION NO.

EP 10 16 5665

This annex lists the patent family members relating to the patent documents cited in the above-mentioned European search report.
The members are as contained in the European Patent Office EDP file on
The European Patent Office is in no way liable for these particulars which are merely given for the purpose of information.

16-11-2010

| Patent document cited in search report | | | Publication date | Patent family member(s) | | | Publication date |
|---|---|---|---|---|---|---|---|
| WO 2006076772 | A1 | | 27-07-2006 | EP | 1838206 | A1 | 03-10-2007 |
| | | | | US | 2008137363 | A1 | 12-06-2008 |
| US 6294775 | B1 | | 25-09-2001 | US | 2002139920 | A1 | 03-10-2002 |
| US 5103497 | A | | 07-04-1992 | NONE | | | |
| US 2007273930 | A1 | | 29-11-2007 | AU | 2004314774 | A1 | 11-08-2005 |
| | | | | BR | PI0418235 | A | 17-04-2007 |
| | | | | CA | 2550305 | A1 | 11-08-2005 |
| | | | | CN | 1902660 | A | 24-01-2007 |
| | | | | EP | 1702304 | A1 | 20-09-2006 |
| | | | | FR | 2864631 | A1 | 01-07-2005 |
| | | | | WO | 2005073912 | A1 | 11-08-2005 |
| | | | | JP | 2007516760 | T | 28-06-2007 |
| | | | | KR | 20060131828 | A | 20-12-2006 |
| US 2009059359 | A1 | | 05-03-2009 | DE | 102007041439 | A1 | 05-03-2009 |
| | | | | EP | 2030560 | A2 | 04-03-2009 |
| | | | | JP | 2009106729 | A | 21-05-2009 |
| US 4154502 | A | | 15-05-1979 | CA | 1141857 | A1 | 22-02-1983 |
| | | | | DE | 2849336 | A1 | 02-08-1979 |
| | | | | GB | 2013924 | A | 15-08-1979 |
| | | | | HK | 17685 | A | 22-03-1985 |
| | | | | JP | 1396998 | C | 24-08-1987 |
| | | | | JP | 54107746 | A | 23-08-1979 |
| | | | | JP | 62003926 | B | 28-01-1987 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4618884 A

- JP 60053919 A